# EUROPÄISCHE PATENTSCHRIFT

(11) **EP 1 123 912 B1**
(45) Veröffentlichungstag und Bekanntmachung des Hinweises auf die Patenterteilung: **20.08.2003**
(21) Anmeldenummer: 01101656.5
(22) Anmeldetag: 29.01.2001
(51) Int. Cl.: C07C 39/04, C07C 37/86, C07C 39/16, C08G 64/20

(54) **Verfahren zur Entfernung von gelöstem Sauerstoff aus Phenol**
Process for eliminating dissolved oxygen from phenol
Procédé pour l'élimination de l'oxygène dissout du phénol

(30) Priorität: 10.02.2000 DE 10005770
(43) Veröffentlichungstag der Anmeldung: 16.08.2001
(73) Patentinhaber: BAYER AG, 51368 Leverkusen (DE)
(72) Erfinder: Heydenreich, Frieder, Dr., 40593 Düsseldorf (DE); Wagner, Rudolf, Dr., 51061 Köln (DE); Bödiger, Michael, Dr., 77573 Texas (US)

(56) Entgegenhaltungen:
- EP-A- 0 526 964
- US-A- 3 437 699
- DATABASE WPI Week 199320, Derwent Publications Ltd., London, GB; Class E14, AN 1993-164394 & JP 5 097 742 A (CHIYODA CORP) 20 April 1993

## Beschreibung

Die Erfindung betrifft ein Verfahren zur Entfernung von gelöstem Sauerstoff aus Phenol durch die Zumischung von Wasserstoff in das Phenol, das dann über Metalle der Platingruppe die auf Trägern aufgebracht sind, geleitet wird, um die Reaktion von Wasserstoff und Sauerstoff zu Wasser gemäß der Formel: 2H₂ + O₂ → 2H₂O zu katalysieren. Die Erfindung betrifft weiterhin Polycarbonat und Bisphenol A, die aus sauerstofffreiem Phenol, hergestellt durch Zumischen von Wasserstoff und anschließendes Leiten über Platinmetall dotierte Ionenaustauscher, hergestellt werden.

Bei der Herstellung des Kunstoffs Polycarbonat ist Phenol ein wichtiger Baustein. Zuerst wird Phenol mit Aceton unter der Einwirkung von Säure zu Bisphenol A umgesetzt. Das molare Verhältnis der Reaktanten Phenol : Aceton liegt dabei im Bereich 8 : 1 bis 14 : 1, bevozugt im Bereich 12:1. In dem folgenden Schritt wird Bisphenol A dann mit entweder Phosgen oder Diphenylcarbonat zu Polycarbonat umgesetzt.

Dieser Kunstoff wird in einem sehr breit gefächerten Anwendungsspektrum eingesetzt, u.a., auch in sehr anspruchsvollen Bereichen wie beispielsweise bei der Herstellung von hochwertiger Optik, von Compact Disks und in der Elektronik.

Häufig verhindern jedoch Verfärbungen in den Kunstoffen den Einsatz in diesen Anwendungen, obgleich ein sehr hoher Aufwand betrieben wird, die Qualität der Einsatzstoffe auf hohem Niveau zu halten.

Wir haben nun überraschenderweise festgestellt, dass sich die Verfärbungen nahezu vollständig vermeiden lassen, wenn Phenol mit Wasserstoff versetzt und dann über einen Träger, bevorzugt Ionenaustauscher, geleitet wird, der mit mindestens einem Metall der Platingruppe dotiert sind ist.

Wie die Verfärbungen zustandekommen ist nicht bis ins Detail geklärt. Im Augenblick wird angenommen, dass sie durch die unselektive Einwirkung von in den Einsatzstoffen vorhandenem Sauerstoff entstehen.

Der Einsatz von mit Edelmetallen der Platingruppe dotierten Anionenaustauschern als Katalysatoren zur katalytischen Sauerstoffentfernung aus Wasser, beispielsweise für den Einsatz in der Dampferzeugung ist seit längerem bekannt und wird bereits weltweit praktiziert (JP-A 58 079 590, CN-A 1 098 384). Dabei wird Wasserstoff dem Wasser stöichiometrisch so zugeführt, so dass der vorhandene Sauerstoff nahezu quantitativ abreagiert wird. Restsauerstoffwerte von weniger als 0,1% des Zulaufwerts sind auf diese Weise erhältlich.

US 3 437 699 beschreibt die Reinigung von Phenol mittels Wasserstoff und Hydrierungskatalysatoren um Verunreinigungen aus dem Phenol zu entfernen. Die vorliegende Erfindung betrifft ein Verfahren zur katalytischen Entfernung von gelöstem Sauerstoff aus Phenol, dadurch gekennzeichnet, dass man
a) dem Phenol Wasserstoff zumischt und
b) den Phenolstrom über mit Platinmetallen dotierte Ionenaustauscher leitet um die Reaktion 2H₂ + O₂ → 2H₂O zu katalysieren.

Die erfindungsgemäß zu verwendenden Platinmetalle sind die Elemente der Reihe Ruthenium, Rhodium, Palladium, Osmium, Iridium, Platin. Für das erfindungsgemäße Verfahren werden Palladium und Platin bevorzugt.

Die erfindungsgemäß zu verwendenden Ionenaustauscher sind bevorzugt Anionenaustauscher. Sie können schwach und/oder stark basische Gruppen enthalten. Insbesondere bevorzugt werden stark basische Anionenaustauscher in der Cl-Form oder schwachbasische Anionenaustauscher in der freien Base-Form. Als Basispolymerisat dient ein vernetztes Polymerisat ethylenisch ungesättigter Monomerer. Beispiele ethylenisch einfach ungesättigter Monomerer sind beispielsweise Styrol, Vinyltoluol, Ethylstyrol, α-Methylstyrol und deren im Kern halogenierte Derivate wie Chlorstyrol; ferner Vinylbenzylchlorid, Acrylsäure, ihre Salze und ihre Ester, besonders der Methyl- und Ethylester, Methacrylsäure, ihre Salze und ihre Ester, besonders der Methylester, ferner die Nitrile und Amide von Acryl- und Methacrylsäure.

Die Polymerisate sind vernetzt - vorzugsweise durch Copolymerisation mit vernetzenden Monomeren mit mehr als einer, vorzugsweise mit 2 oder 3, copolymerisierbaren C=C-Doppelbindungen pro Molekül.

Solche vernetzenden Monomeren umfassen beispielsweise polyfunktionelle Vinylaromaten wie Di- oder Trivinylbenzole, Divinylethylbenzol, Divinyltoluol, Divinylxylol, Divinylethylbenzol, Divinylnaphthalin, polyfunktionelle Allylaromaten wie Dioder Triallylbenzole, polyfunktionelle Vinyl- oder Allylheterocyclen wie Trivinyl- oder Triallylcyanurat oder Trivinyl- oder Triallylisocyanurat, N,N'-C₁- C₆-Alkylendiacrylamide oder -dimethacrylamide wie N,N'-Methylendiacrylamid oder -dimethacrylamid, N,N'-Ethylendiacrylamid oder -dimethacrylamid, Polyvinyl- oder Polyallylether gesättigter C₂-C₂₀-Polyole mit 2 bis 4 OH-Gruppen pro Molekül, wie beispielsweise Ethylenglykoldivinyl- oder -diallylether oder Diethylenglykoldivinyl- oder -diallylether, Ester ungesättigter C₃-C₁₂-Alkohole oder gesättigter C₂-C₂₀-Polyole mit 2 bis 4 OH-Gruppen pro Molekül wie Allylmethacrylat, Ethylenglykoldi(meth)acrylat, Glycerintri(meth)acrylat, Pentaerythrittetra(meth)acrylat, Divinylethylenharnstoff, Divinylpropylenharnstoff, Divinyladipat, aliphatische oder cycloaliphatische Olefine mit 2 oder 3 isolierten C=C-Doppelbindungen wie Hexadien-1,5, 2,5-Dimethylhexadien-1,5, Octadien-1,7, 1,2,4-Trivinylcyclohexan. Als vernetzende Monomere haben sich Divinylbenzol (als Isomerengemisch) sowie Mischungen aus Divinylbenzol und aliphatischen C₆-C₁₂-Kohlenwasserstoffen mit 2 oder 3 C=C-Doppelbindungen besonders bewährt. Die vernetzenden Monomeren werden im allgemeinen in Mengen von 2 bis 20 Gew.-%, vorzugsweise 2 bis 12 Gew.-%, bezogen auf die Gesamtmenge der eingesetzten polymerisierbaren Monomeren, eingesetzt.

Die vernetzenden Monomeren müssen nicht in reiner Form, sondern können auch in Form ihrer technisch gehandelten Mischungen minderer Reinheit (wie z.B. Divinylbenzol in Mischung mit Ethylstyrol) eingesetzt werden.

Die vernetzten Polymerisate können auf bekannte Weise zu Anionenaustauschern weiterverarbeitet werden. Die Anionenaustauscher können einerseits durch Chlormethylierung (vgl. US-A 2 642 417, US-A 2 960 480, US-A 2 597 492, US-A 2 597 493, US-A 3 311 602 und US-A 2 616 877), vorzugsweise mit Chlormethylether, und anschließender Aminierung (vgl. US-A 2 632 000, US-A 2 616 877, USA 2 642 417, US-A 2 632 001 und US-A 2 992 544) mit Ammoniak, einem primären Amin wie Methyl- oder Ethylamin, einem sekundären Amin wie Dimethylamin oder einem tertiären Amin wie Trimethylamin oder Dimethylisopropanolamin bei einer Temperatur von in der Regel 25 bis 150°C hergestellt werden.

Andererseits kann man die Anionenaustauscher nach dem Aminomethylierungsverfahren herstellen, wonach man a) die vernetzten Polymerisate mit Phthalimidderivaten umsetzt und b) die resultierenden Imide hydratisiert. Die Amidomethylierung a) kann durch Umsetzung der vernetzten Polymerisate mit N-Chlormethylphthalimid in Gegenwart von Quellungsmitteln für vernetzte Polymerisate und Friedel-Crafts-Katalysatoren erfolgen (DE-A 1 054 715), wobei das Phthalimid-Derivat in für die gewünschte Substitutionshöhe (0,3 bis 2,0 Substitutionen pro aromatischem Kern) der im vemetzten Polymerisat vorliegenden aromatischen Kerne geeigneten Mengen (oder bis zu 20 %, vorzugsweise bis zu 10 % Überschuß) eingesetzt wird.

Geeignete Quellungsmittel umfassen halogenierte Kohlenwasserstoffe, vorzugsweise chlorierte C₁-C₄-Kohlenwasserstoffe. Das bevorzugteste Quellungsmittel ist 1,2-Dichlorethan.

Bevorzugte Friedel-Crafts-Katalysatoren umfassen beispielsweise AlCl₃, BF₃, FeCl₃, ZnCl₂, TiCl₄, ZrCl₄, SnCl₄, H₃PO₄, HF und HBF₄. Man kann die Katalysatoren in Mengen von 0,01 bis 0,1 Mol pro Mol N-Chlormethylphthalimid einsetzen.

Man kann die Reaktion beispielsweise so durchführen, dass man das vernetzte Polymerisat in eine Lösung von N-Chlormethylphthalimid in einem Quellungsmittel einträgt und die Reaktanden in Gegenwart des Katalysators bei erhöhter Temperatur, in der Regel bei 50 bis 100, vorzugsweise 50 bis 75°C einwirken läßt, bis die Chlorwasserstoff-Entwicklung im wesentlichen beendet ist. Dies ist im allgemeinen nach 2 bis 20 Stunden der Fall. Nach dem Trennen von substituiertem Polymerisat und flüssigem Reaktionsmedium und anorganischen Produkten empfiehlt es sich, das Polymerisat in wäßriger Kochsalzlösung aufzunehmen und Quellungsmittelreste destillativ zu entfernen.

Die Hydrolyse b) des substituierten Polymerisats kann beispielsweise erfolgen, indem man das isolierte Produkt anschließend bei Temperaturen zwischen 100 und 250°C in einem Autoklaven mit ungefähr 5- bis 40 gew.-%iger wäßriger oder alkoholischer Lösung eines Alkalis, wie Natriumhydroxid, Kaliumhydroxid oder mit einer ungefähr 5- bis 50 gew.-%igen wäßrigen Lösung einer Mineralsäure, wie beispielsweise Salzsäure, Bromwasserstoffsäure, Schwefelsäure, verseift. Das Intermediärprodukt kann andererseits auch mit einer 5- bis 50 gew.-%igen wäßrigen oder alkoholischen Lösung von Hydrazinhydrat bei Temperaturen von 50 bis 100°C umgesetzt werden. In bevorzugter Ausführungsform kann die letztgenannte Lösung andere Alkalien, insbesondere kaustische Alkalien, in Mengen von 1 bis 20 Gew.-% enthalten. Das Reaktionsprodukt kann isoliert, mit Wasser gewaschen und anschließend mit einer wäßrigen Lösung von Mineralsäure (vorzugsweise 5 bis 20 gew.-%ig) zur Vervollständigung der Hydrolyse erhitzt werden.

Die erhältlichen Aminoalkylverbindungen können durch Alkylierung abgewandelt werden. Für diesen Zweck dienen bekannte Alkylierungsmittel, wie beispielsweise Methyl-, Ethyl-, Propylchloride sowie -bromide, Dialkylsulfate, Alkylenoxide, Halogenhydrine, Polyhalogenverbindungen, Epihalohydrine, Ethylenimine.

Die vorgenannte Alkylierung der besagten Aminoderivate kann durch Umsatz derselben mit Alkylierungsmitteln in molaren Mengen bei Temperaturen zwischen 20 und 125°C bewirkt werden. Bei Verwendung von beispielsweise Alkylhalogeniden oder Dialkylsulfaten empfiehlt es sich, die zur Neutralisation der gebildeten Halogenwasserstoffsäuren bzw. Alkylschwefelsäuren erforderliche Menge eines alkalischen Mittels wie Natriumhydroxid, Kalciumcarbonat, Magnesiumoxid usw. zuzusetzen. Je nach Menge des angewandten Alkylierungsmittels werden sekundäre, tertiäre oder quaternäre Aminoderivate oder deren Mischungen erhalten. Ein weiteres übliches Alkylierungsmittel stellt die Mischung von Formaldehyd mit Ameisensäure dar, welche in Form einer wäßrigen Lösung, gegebenenfalls in Gegenwart von Mineralsäuren, angewendet wird. Die Reaktion kann mit diesen Alkylierungsmitteln bei Temperaturen zwischen 50 und 120°C durchgeführt werden. In letzterem Fall werden bei Anwendung eines Überschusses an Alkylierungsmittel tertiäre Aminoderivate als alleinige Reaktionsprodukte erhalten. Die tertiären Aminoderivate können vollständig oder teilweise in quaternäre Derivate übergeführt werden, indem weitere Umsetzung mit Alkylierungsmitteln, wie z.B. Methylchlorid, bei Temperaturen zwischen 10 und 120°C erfolgt.

Die verwendeten Anionenaustauscher können gelförmig oder - vorzugsweise - makroporös sein; bevorzugt sind solche auf Polystyrol-Basis. Stark basische Anionenaustauscher in der Cl-Form und schwach basische in der freien Base-Form werden besonders bevorzugt.

Die Dotierung der Anionenaustauscher mit Platinmetallen, bevorzugt Platin oder Palladium kann beispielsweise so erfolgen, dass das Platinmetall, bevorzugt Platin oder Palladium in geeigneter Salzform von den ionenaustauschaktiven Gruppen aufgenommen und anschließend reduziert wird oder es werden reduzierende Substanzen zuerst aufgebracht und das Platinmetall, bevorzugt Platin oder Palladium aus einer geeigneten Lösung anschließend auf dem Harz ausgefällt. Schließlich kann auch kolloidal verteiltes schon reduziertes Platinmetall, bevorzugt Platin oder Palladium aus einer entsprechenden Lösung bzw. Suspension vom Harz adsorptiv aufgenommen werden.

Das in der vorliegenden Erfindung besonders bevorzugte Aufbringverfahren geht von der Salzform des Harzes aus, die zunächst mit einer Palladiumsalzlösung (z.B. Na₂PdCl₄ 2 bis 20 gew.-%ig) behandelt wird, wobei das auf dem Harz befindliche Anion gegen den anionischen Palladiumkomplex ausgetauscht wird. Dabei verteilt sich der Palladiumkomplex vorwiegend im Oberflächenbereich des Harzkorns, so dass vor allem die Bereiche erfaßt werden, die sich durch eine rasche Kinetik auszeichnen.

Die Reduktion des ionogen auf dem Harz gebundenen Edelmetalls, beispielsweise des Palladiums zum metallischen Palladium, kann durch die hierfür gebräuchlichen Reduktionsmittel, wie Hydrazin, Hydroxylamin, Wasserstoff, Ascorbinsäure, Formalin, Ameisensäure in stark alkalischer Lösung bei erhöhter Temperatur erfolgen; bevorzugt wird Hydrazin oder Formalin verwendet.

Die Edelmetall-Gehalte, bevorzugt die Platinmetall-Gehalte, der erfindungsgemäß zu verwendenden Ionenaustauscher liegen im allgemeinen im Bereich von 0,3 bis 10, vorzugsweise 0,5 bis 1,2 g pro Liter Anionenaustauscher.

Aus der DE-A 25 24 722 ist die Verwendung Kupfer- oder Cobaltionen enthaltender Polystyrole zur Reduktion von in Wasser gelöstem Sauerstoff bekannt. Die US-A 4 789 488 empfiehlt Palladium- oder Platin-dotierte Anionenaustauscher zur Verminderung des Sauerstoffgehalts in wäßrigen Systemen mit Wasserstoff. Neben Wasserstoff hat man auch bereits andere Reduktionsmittel, wie z.B. Hydrazin, zur Entfernung von Sauerstoff aus Wasser beschrieben; vergl. F. Martinola et al., VGB Kraftwerkstechnik 64 (1984), S. 61-63. Auch der Einsatz metalldotierter Anionenaustauscher zur gleichzeitigen Entfernung von Sauerstoff und unerwünschten Ionen ist schon diskutiert worden; vergl. F. Martinola, a. a. O.

Es wurde nun festgestellt, dass dieses Verfahren bei der Vermeidung von Verfärbungen in der Polycarbonatproduktion durch die katalytische Reduktion von Sauerstoff sehr effektiv ist. Die oben geschilderte These, dass die Verfärbungen auf Sauerstoffspuren zurückzuführen sind wird dadurch erhärtet. Da Phenol nicht nur die Hauptkomponente des Polycarbonats darstellt, sondern auch bei der Herstellung von Biphenol-A im großen Überschuß vorhanden und am Beginn der Produktionssequenz steht, ist es sinnvoll das Verfahren bei diesem Einsatzstoff anzusetzen.

### Beispiel

Zu einem Phenolstrom von 10m³/h Zulauf zu einen Reaktor zur Herstellung von Bisphenol-A werden 50-100 l/h Wasserstoff eingespeist. Die Phenoltemperatur beträgt 50°C bis 80°C.

Der Systemdruck der Bishenol-A Produktion liegt im Bereich 3 - 10 bar, bei einer Sauerstoffzulaufkonzentration von 0,1 mg/l bis 2 mg/l. Der wasserstoffhaltige Phenolstrom wird durch einen der Bisphenol-A Produktion vorgeschalteten Reaktor, befüllt mit palladisierten schwachbasischen Anionenaustauscher (0,5 m³ Lewatit® Katalysator K3433, Hersteller Bayer AG) geleitet.

Die Harzbetthöhe beträgt 0,5mm, die spezifische Belastung liegt bei 20 Bettvolumen / h und der Druckverlust im Bereich 0,08 - 0,2 bar.
Die gemessene Sauerstoffkonzentration im Ablauf des Katalysatorbetts beträgt nur noch 0,01 bis 0,03 mg/l.

Bei dieser Fahrweise beträgt die Produktverfärbungszahl des Bisphenol-A 13 bis 17 Hazen Schmelzefarbzahl.

Wird die Wasserstoffzufuhr abgeschaltet und der Phenolstrom und der Katalysator umfahren steigt die Produktverfärbungszahl des Bisphenol-A auf >17 Hazen Schmelzefarbzahl.

Entsprechend verändert sich der "Yellowness-Index" des Polycarbonats von ≤1,7 mit nach erfindungsgemäßem Verfahren behandelten Phenol zu ≥1,7, ohne die erfindungsgemäße Behandlung.

## Patentansprüche

1. Verfahren zur katalytischen Entfernung von gelöstem Sauerstoff aus Phenol, **dadurch gekennzeichnet, dass** man
a) dem Phenol Wasserstoff zumischt und
b) den Phenolstrom über mit Platinmetallen dotierte Ionenaustauscher leitet um die Reaktion 2H₂ + O₂ → 2H₂O zu katalysieren.

2. Verfahren gemäß Anspruch 1, **dadurch gekennzeichnet, dass** als Platinmetalle Platin oder Palladium eingesetzt werden.

3. Verfahren gemäß Anspruch 1, **dadurch gekennzeichnet, dass** als Ionenaustauscher Anionenaustauscher mit schwach und/oder stark basischen Gruppen eingesetzt werden.

4. Verfahren gemäß Anspruch 3, **dadurch gekennzeichnet, dass** stark basische Anionenaustauscher in der Cl-Form oder schwachbasische Anionenaustauscher in der freien Base-Form eingesetzt werden.

5. Verfahren gemäß Anspruch 1, **dadurch gekennzeichnet, dass** gelförmige oder makroporäse Ionenaustauscher eingesetzt werden.

6. Verfahren gemäß Anspruch 1, **dadurch gekennzeichnet, dass** der Ionenaustauscher Platinmetall in einer Menge von 0,3 bis 10 g pro Liter Ionenaustauscher enthält.

## Claims

1. Process for the catalytic removal of dissolved oxygen from phenol, **characterized in that**
a) hydrogen is admixed with the phenol and
b) the phenol stream is passed over ion exchangers doped with platinum metals in order to catalyse the reaction 2H₂ + O₂ → 2H₂O.

2. Process according to Claim 1, **characterized in that** platinum or palladium are employed as the platinum metals.

3. Process according to Claim 1, **characterized in that** anion exchangers with weakly and/or strongly basic groups are employed as the ion exchangers.

4. Process according to Claim 3, **characterized in that** strongly basic anion exchangers in the Cl form or weakly basic anion exchangers in the free base form are employed.

5. Process according to Claim 1, **characterized in that** ion exchangers in gel form or macroporous ion exchangers are employed.

6. Process according to Claim 1, **characterized in that** the ion exchanger comprises platinum metal in an amount of 0.3 to 10 g per litre of ion exchanger.

## Revendications

1. Procédé d'élimination catalytique d'oxygène dissous depuis le phénol, **caractérisé en ce que**
a) on mélange de l'hydrogène au phénol, et
b) on fait passer le courant de phénol sur une résine échangeuse d'ions dopée avec un métal de type platine, pour catalyser la réaction 2H₂ + O₂ → 2H₂O.

2. Procédé suivant la revendication 1, **caractérisé en ce que** l'on met en oeuvre le platine ou le palladium comme métal de type platine.

3. Procédé suivant la revendication 1, **caractérisé en ce que** l'on met en oeuvre un échangeur d'anions avec des groupes basiques faibles et/ou forts comme résine échangeuse d'ions.

4. Procédé suivant la revendication 3, **caractérisé en ce que** l'on met en oeuvre un échangeur d'anions fortement basique sous la forme Cl ou un échangeur d'anions faiblement basique sous la forme base libre.

5. Procédé suivant la revendication 1, **caractérisé en ce que** l'on met en oeuvre une résine échangeuse d'ions sous forme de gel ou macroporeuse.

6. Procédé suivant la revendication 1, **caractérisé en ce que** la résine échangeuse d'ions contient un métal du type platine en une quantité allant de 0,3 à 10 g par litre de résine échangeuse d'ions.
